# EUROPEAN PATENT APPLICATION

(11) **EP 4 755 806 A1**
(43) Date of publication of application: **10.06.2026**
(21) Application number: 24849130.0
(22) Date of filing: 29.07.2024
(51) Int. Cl.: B64G 1/64, B64G 4/00

(54) **CAPTURING SYSTEM AND CAPTURING METHOD**

(30) Priority: 31.07.2023 US 202363516816 P; 03.08.2023 US 202363517570 P
(71) Applicant: Astroscale Japan Inc., Tokyo, 130-0013 (JP)
(72) Inventor: IWAI, Takashi, Tokyo 130-0013 (JP); NISHIDA, Shin-ichiro, Tokyo 130-0013 (JP)
(74) Representative: Plasseraud IP
(86) International application number: PCT/JP2024/026950
(87) International publication number: WO 2025/028479

(57) **Abstract**

Provided is a capturing system 1 that can safely capture an object to be captured that has an annular opening portion O_{T} and exhibits a residual motion with a simple configuration and operation. The capturing system 1 includes a supporting body 10 attached to a spacecraft 2 moving in the outer space, a plurality of deployable rods 20 configured to be radially deployed from the supporting body 10, and a gripping mechanism 30 attached to each of the plurality of deployable rods 20, and configured to be coupled to an annular opening portion O_{T} of the object to be captured. The gripping mechanism 30 includes a first link 31 pivotable about an axis 23 provided at a tip end of each of the plurality of deployable rods 20, and configured to be deployed to a position spaced away from the supporting body 10, and a second link 32 pivotable about the axis 23, and configured to change an angle with respect to the first link 31.

## Description

### Technical Field

The present invention relates to a capturing system and a capturing method.

### Background Art

Currently, a technology has been proposed related to a service satellite configured to approach a non-cooperative space object (for example, space debris such as an artificial satellite that has run out of fuel or a rocket upper stage that has finished the role) that does not have an interface such as a marker, and to provide on-orbit services or to remove debris. It is known that such a service satellite needs to have a function of being coupled to a cylindrical or conical portion, which is a characteristic configuration of the object, in order to capture the non-cooperative space object (an artificial satellite, a rocket upper stage, or the like).

Therefore, in recent years, a debris capture device including a base frame, a plurality of expansion and contraction devices provided on the base frame, and a V-shaped end effector coupled to each of the plurality of expansion and contraction devices has been proposed (refer to Patent Literature 1). It is said that, by using such a device, a plurality of end effectors is deployed radially outward, and each end effector is locked from the inside to an end part (annular opening portion) of a cylindrical or conical portion of a rocket upper stage, which is an object to be captured, whereby the rocket upper stage can be captured.

### Citation List

### Patent Literature

Patent Literature 1: Japanese Patent No. 7389991

### Summary of Invention

### Technical Problem

Incidentally, the non-cooperative space object as a capture target often exhibits a residual motion such as a rotational motion or precession, and it is necessary to mitigate a load caused by such a residual motion when coupling or capturing by the service satellite. In addition, there is a problem in that it is difficult to perform position or attitude control with high accuracy for the object to be captured that exhibits the residual motion. In this regard, in the device in the related art disclosed in Patent Literature 1, since the residual motion of the rocket upper stage as the object to be captured is not taken into account, it is difficult to perform alignment of the rocket upper stage with respect to the annular opening portion, and a large service satellite having a complex system configuration or operation scenario with a high-level attitude and orbit control system or propulsion system is required to solve the above problem. In addition, when a malfunction occurs during the capture operation and a collision or hooking occurs between the service satellite and the capture targets, a situation in which components are damaged and scattered in various orbits may occur, which may lead to further problems on the orbit such as debris of the service satellite. Accordingly, in order to provide a low-cost on-orbit service, there has been a demand for the development of a technology for safely capturing an object to be captured that exhibits a residual motion with a simple configuration and operation.

The present invention has been made in view of such circumstances, and an object of the present invention is to provide a capturing system that can safely capture an object to be captured having an annular opening portion and exhibiting a residual motion with a simple configuration and operation.

### Solution to Problem

In order to achieve the object, according to a first aspect of the present invention, there is provided a capturing system that captures an object to be captured existing in an outer space, the capturing system including: a supporting body attached to a spacecraft moving in the outer space; a plurality of deployable rods configured to be radially deployed from the supporting body; and a gripping mechanism attached to each of the plurality of deployable rods, and configured to be coupled to an annular opening portion of the object to be captured, wherein the gripping mechanism includes a first link pivotable about a first axis provided at a tip end of each of the plurality of deployable rods, and configured to be deployed to a position spaced away from the spacecraft, and a second link pivotable about the first axis (or a second axis disposed in a vicinity of the first axis), and configured to change an angle with respect to the first link.

When such a configuration is adopted, the first link of the gripping mechanism attached to each of the plurality of deployable rods radially deployed from the supporting body attached to the spacecraft is pivoted about the first axis provided at the tip end of each deployable rod and is deployed to the position spaced away from the supporting body, so that each of the first links may be brought into a state where each of the first links can abut on the annular opening portion of the object to be captured existing in the outer space (for example, each of the first links may be disposed on the same imaginary plane). Accordingly, when the spacecraft approaches the object to be captured, each of the first links may abut on the annular opening portion of the object to be captured. In addition, the second link of the gripping mechanism is configured to be pivotable about the first axis (or the second axis disposed in the vicinity of the first axis) to change the angle with respect to the first link. Therefore, when the first link abuts on the annular opening portion of the object to be captured, the second link may be inserted into the inside of the annular opening portion of the object to be captured by setting the angle of the second link with respect to the first link to an obtuse angle. In this case, even when the center position of the supporting body is deviated from the center position of the annular opening portion of the object to be captured, the center position of the supporting body may be brought close to the center position of the annular opening portion of the object to be captured by a guide structure having a substantially conical shape formed by each of the second links forming an obtuse angle attitude with respect to the first link (centering function). In this manner, after the second link is inserted into the inside of the annular opening portion of the object to be captured, the angle of the second link with respect to the first link is set to an acute angle, so that the annular opening portion may be gripped by sandwiching the annular opening portion of the object to be captured between the first link and the second link. As a result, the gripping mechanism may be coupled to the object to be captured. Accordingly, even when the object to be captured exhibits the residual motion, it is possible to safely capture the object to be captured with a simple configuration and operation.

In the capturing system according to the present invention, the gripping mechanism may include a third link pivotable about a third axis provided at a tip end of each of the second links, and configured to change an angle with respect to the second link.

When such a configuration is adopted, the third link may be hooked onto the annular opening portion of the object to be captured, when the annular opening portion of the object to be captured is sandwiched between the first link and the second link. Accordingly, the gripping mechanism may be reliably coupled to the object to be captured.

In addition, in the capturing system according to the present invention, the supporting body may be attached to the spacecraft via a robot arm. In such a case, a buffer elastic body may be provided at a connection part between the supporting body and the robot arm or inside the robot arm.

When such a configuration is adopted, the buffer elastic body provided at the connection part between the supporting body and the robot arm or inside the robot arm can suppress the transmission of the shocking load generated when the gripping mechanism is coupled to the object to be captured and the object to be captured is captured, to the spacecraft.

In addition, in the capturing system according to the present invention, three or more of the plurality of deployable rods may be provided, and the three or more deployable rods may be configured to be radially deployed in different directions from the supporting body.

When such a configuration is adopted, the three or more deployable rods are radially deployed in different directions from the supporting body, and the gripping mechanism attached to each of the deployable rods may be coupled to the annular opening portion of the object to be captured. Accordingly, since the gripping mechanism may be coupled to at least three places of the annular opening portion of the object to be captured, it is possible to more reliably capture the object to be captured.

According to a second aspect of the present invention, there is provided a capturing method that captures an object to be captured existing in an outer space using the capturing system according to the first aspect, the capturing method including: a deployment step of radially deploying the plurality of deployable rods from the supporting body of the capturing system; and a coupling step of coupling the gripping mechanism attached to each of the plurality of deployable rods to the object to be captured, in which the coupling step includes an abutment step of causing each of the first links of the gripping mechanism to abut on the annular opening portion of the object to be captured by pivoting each of the first links about an axis provided at the tip end of each of the plurality of deployable rods and deploying each of the first links to the position spaced away from the supporting body, when the spacecraft approaches the object to be captured, an inserting step of inserting the second link of the gripping mechanism into an inside of the annular opening portion by setting an angle of the second link with respect to the first link to an obtuse angle, when the first link abuts on the annular opening portion, and a gripping step of gripping the annular opening portion by sandwiching the annular opening portion between the first link and the second link by setting the angle of the second link with respect to the first link to an acute angle, after the second link is inserted into the inside of the annular opening portion.

When such a method is adopted, the first link of the gripping mechanism attached to each of the plurality of deployable rods radially deployed from the supporting body is pivoted about the axis provided at the tip end of each deployable rod and is deployed to the position spaced away from the supporting body, so that each of the first links may abut on the annular opening portion of the object to be captured when the spacecraft approaches the object to be captured. In this case, by setting the angle of the second link with respect to the first link to an obtuse angle, the second link may be inserted into the inside of the annular opening portion of the object to be captured. Thereafter, by setting the angle of the second link with respect to the first link to an acute angle, the annular opening portion of the object to be captured may be gripped by sandwiching the annular opening portion between the first link and the second link. As a result, the gripping mechanism may be coupled to the object to be captured. Accordingly, even when the object to be captured exhibits the residual motion, it is possible to safely capture the object to be captured with a simple configuration and operation.

In the capturing method according to the present invention, in the abutment step, each of the first links may be disposed on the same imaginary plane by pivoting each of the first links about the axis and deploying each of the first links to the position spaced away from the supporting body.

When such a method is adopted, since each of the first links may be disposed on the same imaginary plane (a pseudo plane is formed by each of the first links), there is an advantage that each of the first links is likely to abut on the annular opening portion of the object to be captured.

In the capturing method according to the present invention, the coupling step may include a centering step of bringing a center position of the supporting body close to a center position of the annular opening portion of the object to be captured by a guide structure having a substantially conical shape formed by each of the second links forming an obtuse angle attitude with respect to the first link.

When such a method is adopted, even when the center position of the supporting body is deviated from the center position of the annular opening portion of the object to be captured, the center position of the supporting body may be brought close to the center position of the annular opening portion of the object to be captured by the guide structure having a substantially conical shape formed by each of the second links forming the obtuse angle attitude with respect to the first link.

### Advantageous Effect of Invention

According to the present invention, it is possible to provide a capturing system capable of safely capturing an object to be captured that has an annular opening portion and exhibits a residual motion with a simple configuration and operation.

### Brief Description of Drawings

[Figure 1] Figure 1 is a diagram illustrating a configuration of a capturing system according to an embodiment of the present invention.
[Figure 2] Figure 2 is a diagram illustrating a state in which a deployable rod of the capturing system illustrated in Figure 1 is folded (a state during launching).
[Figure 3] Figure 3 is a diagram illustrating an initial deployment state of the deployable rod of the capturing system illustrated in Figure 1.
[Figure 4] Figure 4 is a diagram illustrating a mid-deployment state of the deployable rod of the capturing system illustrated in Figure 1.
[Figure 5] Figure 5 is a diagram illustrating a final deployment state of the deployable rod of the capturing system illustrated in Figure 1 (a state in which a first link of a gripping mechanism abuts on an annular opening portion of an object to be captured, and a second link is inserted into the annular opening portion of the object to be captured).
[Figure 6] Figure 6 is a diagram illustrating a state in which the second link of the gripping mechanism of the capturing system illustrated in Figure 1 forms an acute angle with respect to the first link.
[Figure 7] Figure 7 is a diagram illustrating a state in which the annular opening portion of the object to be captured is gripped by the gripping mechanism of the capturing system illustrated in Figure 1.
[Figure 8] Figure 8 is a diagram illustrating an example of a configuration of a buffer mechanism of the capturing system illustrated in Figure 1.
[Figure 9] Figure 9 is a diagram illustrating another example of a configuration of a buffer mechanism of the capturing system illustrated in Figure 1.
[Figure 10] Figure 10 is a flowchart illustrating a method of capturing the object to be captured using the capturing system illustrated in Figure 1.
[Figure 11] Figure 11 is a diagram illustrating an inserting step of a capturing method using the capturing system illustrated in Figure 1.
[Figure 12] Figure 12 is a diagram illustrating a method of capturing the object to be captured using a third link of the capturing system illustrated in Figure 1.

### Description of Embodiments

Hereinafter, an embodiment of the present invention will be described with reference to the drawings.

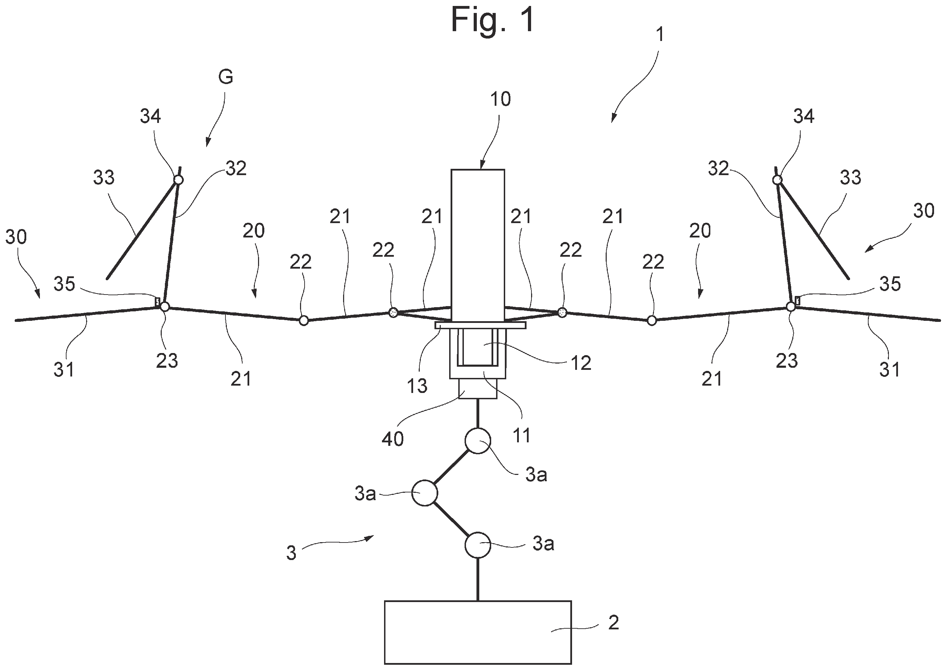

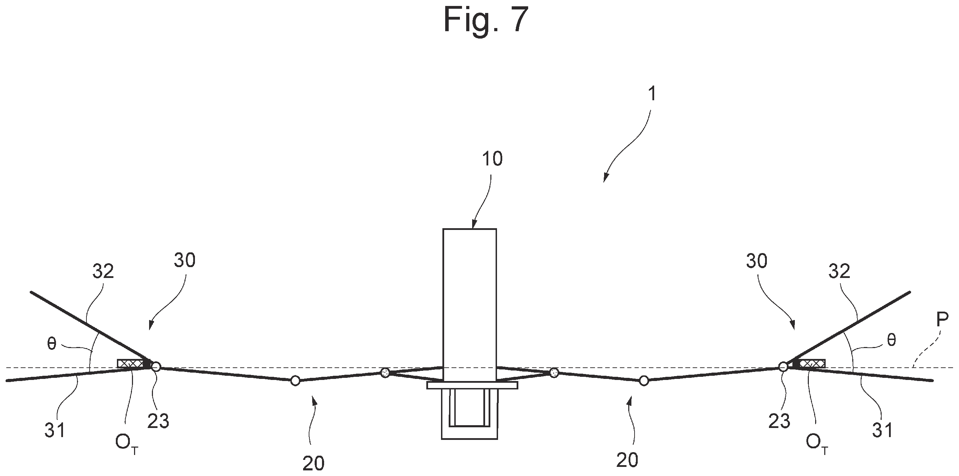

First, a configuration of a capturing system 1 according to an embodiment of the present invention will be described with reference to Figures 1 to 9. The capturing system 1 functions to capture an object to be captured existing in an outer space. The object to be captured in the present embodiment is a non-cooperative space object (an artificial satellite or a rocket upper stage) that exhibits a residual motion, such as a rotational motion and precession, and has an annular opening portion O_{T} (refer to Figure 7) that is an end part of a cylindrical or conical portion.

As illustrated in Figure 1, the capturing system 1 includes a supporting body 10 attached to a spacecraft 2 that moves in the outer space via a robot arm 3 and a buffer mechanism 40 (described later), a plurality of deployable rods 20 configured to be radially deployed from the supporting body 10, and a gripping mechanism 30 attached to each of the plurality of deployable rods 20 and configured to be coupled to the annular opening portion O_{T} of an object to be captured.

As illustrated in Figure 1, the supporting body 10 includes a housing-shaped main body 11 having a space for accommodating various components therein, a drive control unit 12 that controls driving of the deployable rod 20, and a locking mechanism 13 that fixes the deployable rod 20 when launching. The three-dimensional shape of the main body 11 is not particularly limited, and for example, shapes such as a cylindrical shape, a prismatic shape, a rectangular parallelepiped shape, or a cubic shape can be adopted. The size of the main body 11 is not particularly limited as long as the main body 11 has a size that can be attached to a rocket and launched into the outer space. The spacecraft 2 or the main body 11 is provided with an object sensor or the like for detecting the object to be captured. The drive control unit 12 is mounted in a space inside the main body 11 and functions to drive and control the operation of the deployable rod 20 and the gripping mechanism 30.

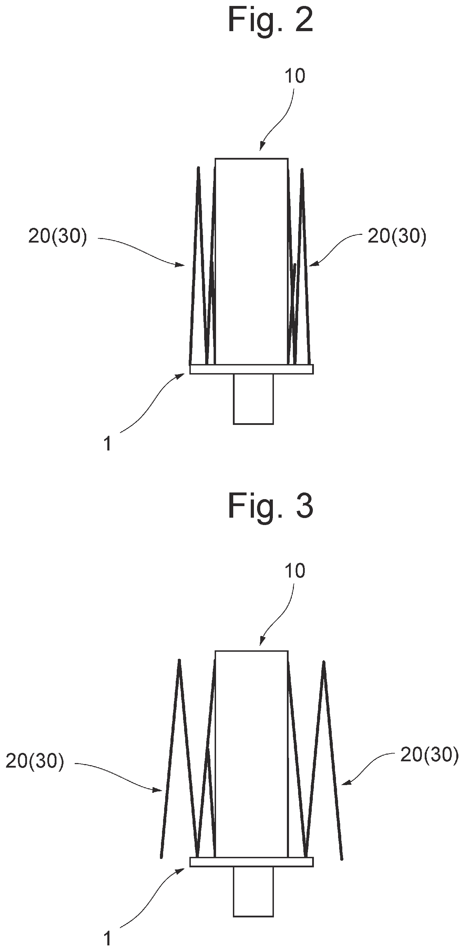

The plurality of deployable rods 20 is configured to be radially deployed in different directions from the main body 11 of the supporting body 10. It is preferable that three or more of the deployable rods 20 are provided, and in the present embodiment, six deployable rods 20 are adopted. Each deployable rod 20 includes a plurality of rod portions 21 and a hinge portion 22 that pivotably connects the rod portions 21, and is configured to be foldable as illustrated in Figure 2 at the time of launching and to be deployed as illustrated in Figures 3 to 7.

The gripping mechanism 30 includes a first link 31 pivotable about an axis 23 provided at a tip end of each of the plurality of deployable rods 20 and configured to deploy to a position spaced away from the supporting body 10, a second link 32 pivotable about the axis 23 and configured to change an angle with respect to the first link 31, and a third link 33 pivotable about an axis 34 provided at a tip end of each of the second links 32 and configured to change an angle with respect to the second link 32. The second link 32 may pivot about an axis (second axis) different from the axis 23 (first axis) serving as the pivot center of the first link 31. In this case, the second axis is disposed in the vicinity of the axis 23 (first axis), and the axis 34 serving as the pivot center of the third link 33 corresponds to the third axis.

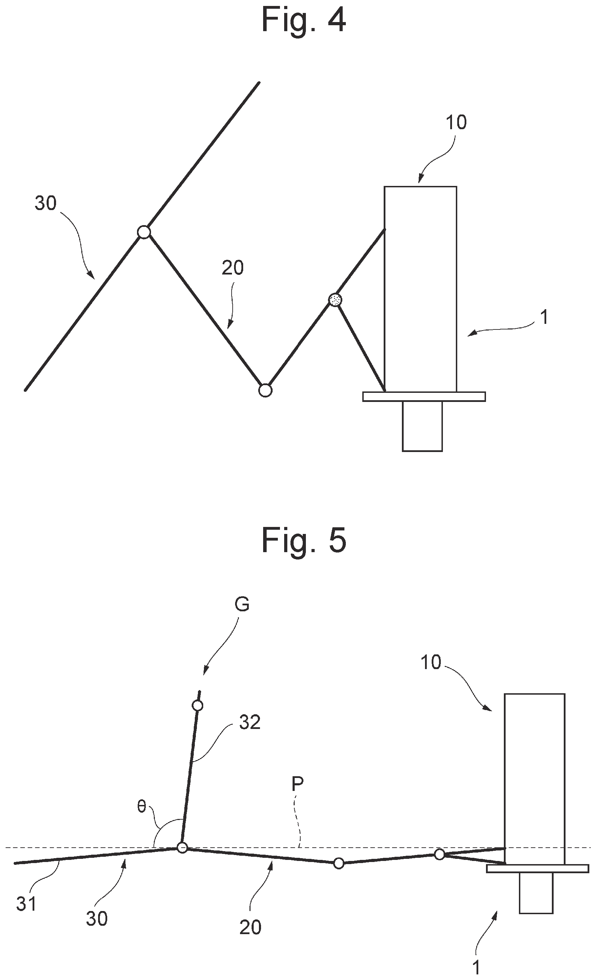

When the spacecraft 2 approaches the object to be captured, the drive control unit 12 can pivot each of the first links 31 of the gripping mechanism 30 about the axis 23 provided at the tip end of each of the plurality of deployable rods 20 to deploy each of the first links 31 to a position spaced away from the supporting body 10, and can dispose each of the first links 31 on the same imaginary plane P as illustrated in Figures 5 and 7 (that is, form a pseudo plane by each of the first links 31). In this state, each of the first links 31 can abut on the annular opening portion O_{T} of the object to be captured.

In addition, when the first link 31 abuts on the annular opening portion O_{T}, the drive control unit 12 sets an angle θ of the second link 32 of the gripping mechanism 30 with respect to the first link 31 to an obtuse angle (refer to Figures 1 and 5), so that the second link 32 can be inserted into the inside of the annular opening portion O_{T}. In this case, a guide structure G (refer to Figures 1 and 5) having a substantially conical shape formed by each of the second links 32 forming an obtuse angle attitude with respect to the first link 31 can bring the center position of the spacecraft 2 close to the center position of the annular opening portion O_{T} of the object to be captured (centering function).

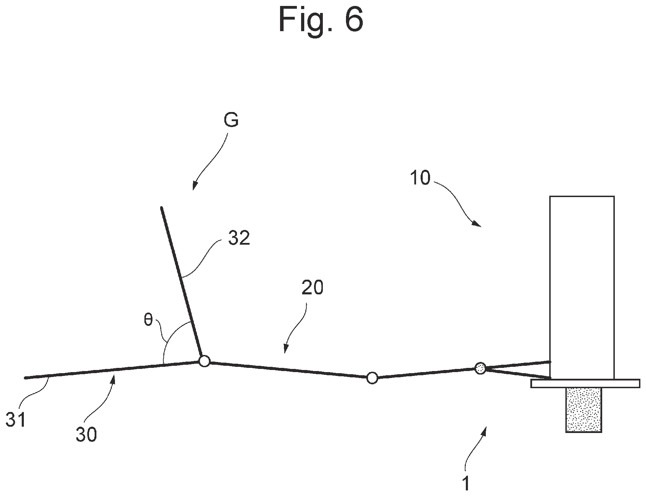

Furthermore, the drive control unit 12 can grip the annular opening portion O_{T} by sandwiching the annular opening portion O_{T} between the first link 31 and the second link 32 by setting the angle θ of the second link 32 with respect to the first link 31 to an acute angle after inserting the second link 32 into the inside of the annular opening portion O_{T} (refer to Figures 6 and 7). The gripping mechanism 30 is provided with a fixed detection sensor 35 (refer to Figure 1) that detects that the annular opening portion O_{T} is gripped by sandwiching the annular opening portion O_{T} between the first link 31 and the second link 32.

In the present embodiment, the buffer mechanism 40 is provided at a connection part between the supporting body 10 and the robot arm 3. As a result, the buffer mechanism 40 can suppress the transmission of a shocking load generated when the gripping mechanism 30 is coupled to the object to be captured and the object to be captured is captured, to the spacecraft 2. In addition, a buffer mechanism can be provided inside the robot arm 3 (for example, inside each joint 3a of the robot arm 3).

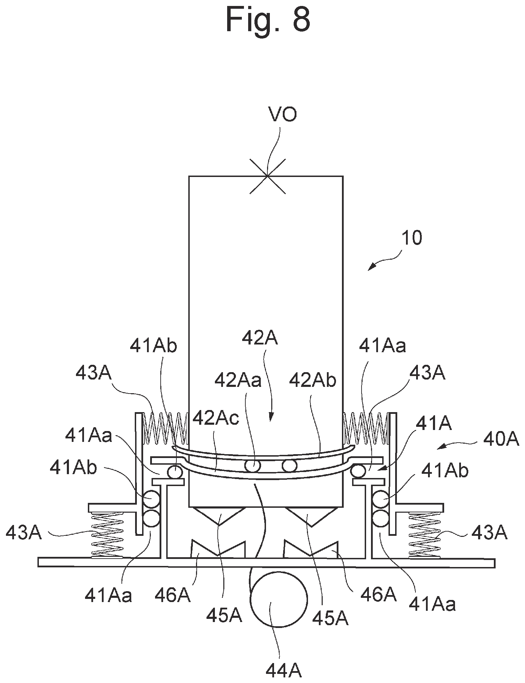

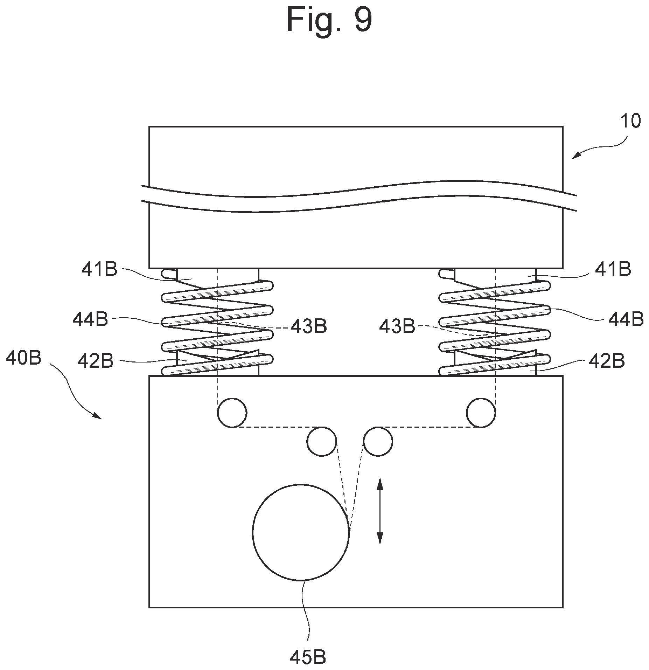

As the buffer mechanism 40, a configuration 40A that enables buffering with a virtual rotation center of an attitude displacement to be set upward by using a movable support mechanism including ball bearings and the like as illustrated in Figure 8, or a configuration 40B that enables the translation and the buffering of the attitude in all axes by an elastic body as illustrated in Figure 9 can be adopted.

A buffer mechanism 40A illustrated in Figure 8 is a mechanism having degrees of freedom of the three translational axes and the three attitude axes, and includes a translational movable portion 41A and an attitude movable portion 42A. The translational movable portion 41A has a linear guide 41Aa and a ball bearing mechanism 41Ab for each of the three translational axes, and supports the support body 10. The attitude movable portion 42A has three or more ball bearings 42Aa, a ball holding retainer 42Ab, and a spherical seat 42Ac, and supports the support body 10 so that the virtual rotation center VO is positioned closer to the tip end of the supporting body 10. An elastic body 43A is provided in each of the axes of the movable portions 41A and 42A, and the supporting body 10 is displaced according to the stiffness of the elastic body when an external force is applied, but the supporting body 10 can be returned to the center position or attitude in a state where the external force is not applied. In addition, the buffer mechanism 40 of Figure 8 can rigidly hold the supporting body 10 during launching or the like by locking a retraction and holding mechanism 44A in a state where the supporting body 10 is displaced to the lower end in the vertical direction by the retraction and holding mechanism 44A using a wire or the like, and holding cone fittings 45A, which are provided at three or more locations in the lower part of the supporting body 10, are fitted with holding cup fittings 46A that are correspondingly provided on a predetermined base (connection portion of the robot arm 3, or the like).

A buffer mechanism 40B illustrated in Figure 9 includes holding cone fittings 41B, which are provided at three or more locations of the lower part of the supporting body 10, and holding cup fittings 42B, which are correspondingly provided on a predetermined base (connection portion of the robot arm 3), and the supporting body 10 and the base are connected to each other by a wire 43B and an elastic body 44B. When the wire 43B is unwound and loosened by a winding mechanism 45B, the supporting body 10 can be displaced with respect to the base in the three translational axes direction and the three attitude axes. On the other hand, the supporting body 10 can be rigidly held during the launching or the like by locking the winding mechanism 45B in a state where the supporting body 10 is displaced to the lower end in the vertical direction and the holding cone fitting 41B and the holding cup fitting 42B are fitted to each other.

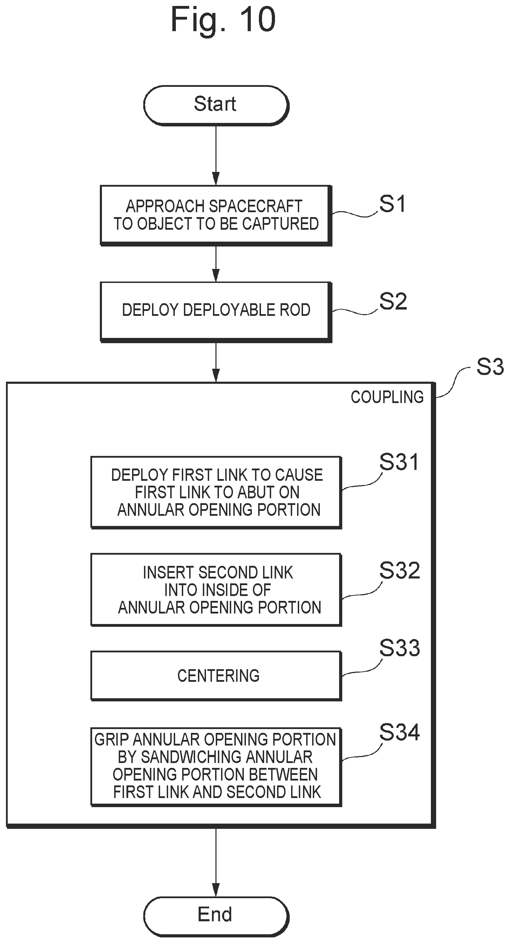

Next, a method of capturing an object to be captured existing in the outer space by the capturing system 1 according to the present embodiment will be described with reference to the flowchart of Figure 10, and the like.

First, the spacecraft 2 is moved toward the object to be captured, and the supporting body 10 attached to the spacecraft 2 approaches the object to be captured (approaching step: S1). In this case, the spacecraft 2 searches for the object to be captured while performing sensing by the object sensor or the like. For example, the search can be realized through a process in which light emitted from a light projector attached to the spacecraft 2 is reflected by a characteristic optical surface or a surface of a marker of the object to be captured, the light is captured by the object sensor, and a computing unit recognizes the light, or the like. The spacecraft 2 can move to approach the object to be captured by recognizing the relative position and the relative attitude of the object to be captured by using information obtained by recognizing the characteristic optical surface or the marker pattern, or the like. In this manner, while the spacecraft 2 is approaching the object to be captured, the deployable rod 20 is in a folded state as illustrated in Figure 2.

Next, in a state in which the distance between the object to be captured and the supporting body 10 is equal to or less than a predetermined value, as illustrated in Figures 3 to 5, the plurality of deployable rods 20 is radially deployed from the supporting body 10 of the capturing system 1 (deployment step: S2). Subsequently, the gripping mechanism 30 attached to each of the plurality of deployable rods 20 is coupled to the object to be captured (coupling step: S3).

Here, the coupling step S3 will be described in detail.

The coupling step S3 includes an abutment step S31 of causing each of the first links 31 of the gripping mechanism 30 to abut on the annular opening portion O_{T} of the object to be captured by pivoting each of the first links 31 about the axis 23 provided at a tip end of each of the plurality of deployable rods 20 and deploying each of the first links 31 to a position spaced away from the supporting body 10, when the spacecraft 2 (or the supporting body 10 by the moving operation of the robot arm 3) approaches the object to be captured. In the abutment step S31, each of the first links 31 can be disposed on the same imaginary plane P as illustrated in Figure 5 (that is, a pseudo plane is formed by each of the first links 31) by pivoting each of the first links 31 about the axis 23 and deploying each of the first links 31 to a position spaced away from the supporting body 10.

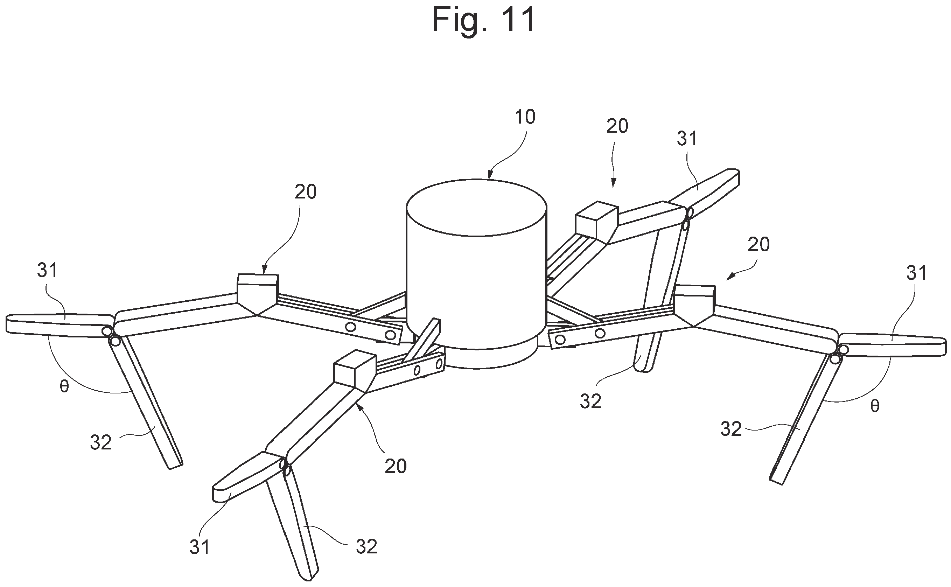

In addition, the coupling step S3 includes an inserting step S32 of inserting the second link 32 of the gripping mechanism 30 into the inside of the annular opening portion O_{T} by setting the angle θ of the second link 32 with respect to the first link 31 to an obtuse angle as illustrated in Figures 5 and 11, when the first link 31 abuts on the annular opening portion O_{T} (Figure 11 is obtained by reversing the vertical relationship with respect to Figure 5, and the object to be captured is disposed at the bottom of the paper surface). In addition, the coupling step S3 also includes a centering step S33 of bringing the center position of the supporting body 10 close to the center position of the annular opening portion O_{T} of the object to be captured by the guide structure G (refer to Figures 1 and 5) having a substantially conical shape formed by each of the second links 32 forming an obtuse angle attitude with respect to the first link 31.

Furthermore, the coupling step S3 includes a gripping step S34 of gripping the annular opening portion O_{T} by sandwiching the annular opening portion O_{T} between the first link 31 and the second link 32 by setting the angle θ of the second link 32 with respect to the first link 31 to an acute angle as illustrated in Figures 6 and 7, after the second link 32 is inserted into the inside of the annular opening portion O_{T}. Although the third link 33 is not illustrated in Figures 2 to 7, when the first link 31 and the second link 32 sandwich the annular opening portion O_{T} of the object to be captured in the gripping step S34, the third link 33 can also be hooked onto the annular opening portion O_{T} of the object to be captured.

By the step groups S1 to S3, the gripping mechanism 30 of the capturing system 1 is coupled to the annular opening portion O_{T} of the object to be captured, and the object to be captured can be captured. In the capturing method in the present embodiment, since the first link 31 that forms a diameter larger than the annular opening portion O_{T} of the object to be captured abuts on the annular opening portion O_{T}, even when a malfunction of position or attitude control occurs during coupling, it is advantageous in terms of safety in that unexpected entry into or hooking onto the annular opening portion O_{T} does not occur. Thereafter, in order to detach the object to be captured, the gripping state may be released by setting the angle θ of the second link 32 with respect to the first link 31, which is an acute angle, to an obtuse angle. In this manner, since the angle θ of the second link 32 with respect to the first link 31 can be set to an obtuse angle as described above, the hooking between the gripping mechanism 30 and the object to be captured is unlikely to occur during the detachment, and a malfunction of the detachment operation is unlikely to occur.

In the capturing system 1 according to the above-described embodiment, the first link 31 of the gripping mechanism 30 attached to each of the plurality of deployable rods 20 radially deployed from the supporting body 10 is deployed to a position spaced away from the supporting body 10 by pivoting the first link 31 about the axis 23 provided at the tip end of each of the deployable rods 20, so that each of the first links 31 can be brought into a state of where each of the first links 31 can abut on the annular opening portion O_{T} of the object to be captured existing in the outer space (each of the first links 31 can be disposed on the same imaginary plane P). Accordingly, when the spacecraft 2 approaches the object to be captured, each of the first links 31 can abut on the annular opening portion O_{T} of the object to be captured. That is, in the present embodiment, since the capturing proceeds by moving the spacecraft 2 (or the supporting body 10 by the moving operation of the robot arm 3) forward, there is an advantage that the required accuracy of the alignment in the front-rear direction (vertical direction) is relaxed. In addition, the second link 32 of the gripping mechanism 30 can be pivoted about the axis 23 to change an angle with respect to the first link 31. Therefore, when the first link 31 abuts on the annular opening portion O_{T} of the object to be captured, the second link 32 can be inserted into the inside of the annular opening portion O_{T} of the object to be captured by setting the angle of the second link 32 with respect to the first link 31 to an obtuse angle (refer to Figure 11 and the like). In this case, even when the center position of the supporting body 10 is deviated from the center position of the annular opening portion O_{T} of the object to be captured, the center position of the supporting body 10 can be brought close to the center position of the annular opening portion O_{T} of the object to be captured by the guide structure G having a substantially conical shape formed by each of the second links 32 that form the obtuse angle attitude with respect to the first link 31 (centering function). Accordingly, in the present embodiment, there is an advantage that the required accuracy of the alignment in the radial direction (horizontal direction) is also relaxed. In this manner, after the second link 32 is inserted into the inside of the annular opening portion O_{T} of the object to be captured, the angle of the second link 32 with respect to the first link 31 is set to an acute angle, so that the annular opening portion O_{T} of the object to be captured can be gripped by sandwiching the annular opening portion O_{T} between the first link 31 and the second link 32. As a result, the gripping mechanism 30 can be coupled to the object to be captured, and the object to be captured can be safely captured with a simple configuration and operation.

In addition, in the capturing system 1 according to the above-described embodiment, when the first link 31 and the second link 32 sandwich the annular opening portion O_{T} of the object to be captured, the third link 33 can be hooked onto the annular opening portion O_{T} of the object to be captured. Accordingly, the gripping mechanism 30 can be reliably coupled to the object to be captured.

In addition, in the capturing system 1 according to the above-described embodiment, the buffer mechanism 40 provided at the connection part between the supporting body 10 and the robot arm 3 (or inside the robot arm 3) can suppress the transmission of the shocking load generated when the gripping mechanism 30 is coupled to the object to be captured and the object to be captured is captured, to the spacecraft 2.

In addition, in the capturing system 1 according to the above-described embodiment, three or more deployable rods 20 are radially deployed in different directions from the supporting body 10, and the gripping mechanism 30 attached to each of the deployable rods 20 can be coupled to the annular opening portion O_{T} of the object to be captured. Accordingly, since the gripping mechanism 30 can be coupled to at least three places of the annular opening portion O_{T} of the object to be captured, it is possible to more reliably capture the object to be captured.

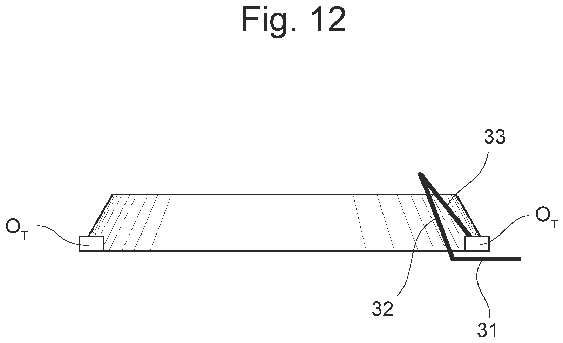

In addition, in the present embodiment, in the gripping step S34 included in the coupling step S3, the example has been described in which the annular opening portion O_{T} is gripped by sandwiching the annular opening portion O_{T} between the first link 31 and the second link 32 by setting the angle θ of the second link 32 with respect to the first link 31 to an acute angle after inserting the second link 32 into the inside of the annular opening portion O_{T}. However, as illustrated in Figure 12, the annular opening portion O_{T} can also be gripped by sandwiching the annular opening portion O_{T} between the first link 31 and the third link 33 by setting the angle of the third link 33 with respect to the second link 32 to an acute angle while maintaining the angle θ of the second link 32 with respect to the first link 31 to an obtuse angle. Such a method is effective when the diameter of the conical portion of the object to be captured is reduced from the annular opening portion O_{T} toward the inside as illustrated in Figure 12.

The present invention is not limited to each of the above-described embodiments, and those embodiments of which the design is appropriately modified by a person skilled in the art are also within the scope of the present invention as long as the modifications have the features of the present invention. In other words, each element and its disposition, material, condition, shape, size, and the like included in the embodiment are not limited to those exemplified, and may be appropriately changed. In addition, each element included in the above-described embodiments is able to be combined as much as technically possible, and the combination of the elements is also included in the scope of the present invention as long as the features of the present invention are included.

### Reference Signs List

1: capturing system
2: spacecraft
3: robot arm
10: supporting body
20: deployable rod
23: axis (first axis)
30: gripping mechanism
31: first link
32: second link
33: third link
34: axis (third axis)
40: buffer mechanism
G: guide structure
O_{T}: annular opening portion of object to be captured
P: imaginary plane
S2: deployment step
S3: coupling step
S31: abutment step
S32: inserting step
S33: centering step
S34: gripping step

## Claims

1. A capturing system that captures an object to be captured existing in an outer space, the capturing system comprising:
a supporting body attached to a spacecraft moving in the outer space;
a plurality of deployable rods configured to be radially deployed from the supporting body; and
a gripping mechanism attached to each of the plurality of deployable rods, and configured to be coupled to an annular opening portion of the object to be captured,
wherein the gripping mechanism includes:
a first link pivotable about a first axis provided at a tip end of each of the plurality of deployable rods, and configured to be deployed to a position spaced away from the supporting body; and
a second link pivotable about the first axis or a second axis disposed in a vicinity of the first axis, and configured to change an angle with respect to the first link.

2. The capturing system according to Claim 1, wherein the gripping mechanism includes a third link pivotable about a third axis provided at a tip end of each of the second links, and configured to change an angle with respect to the second link.

3. The capturing system according to Claim 1 or 2, wherein:
the supporting body is attached to the spacecraft via a robot arm, and
the capturing system further comprises a buffer mechanism provided at a connection part between the supporting body and the robot arm or inside the robot arm.

4. The capturing system according to Claim 1 or 2, wherein three or more of the plurality of deployable rods are provided, and are configured to be radially deployed in different directions from the supporting body.

5. A capturing method that captures an object to be captured existing in an outer space using the capturing system according to Claim 1, the method comprising:
a deployment step of radially deploying the plurality of deployable rods from the supporting body of the capturing system; and
a coupling step of coupling the gripping mechanism attached to each of the plurality of deployable rods to the object to be captured,
wherein the coupling step includes:
an abutment step of causing each of the first links of the gripping mechanism to abut on the annular opening portion of the object to be captured by pivoting each of the first links about an axis provided at the tip end of each of the plurality of deployable rods and deploying each of the first links to the position spaced away from the supporting body, when the spacecraft approaches the object to be captured;
an inserting step of inserting the second link of the gripping mechanism into an inside of the annular opening portion by setting an angle of the second link with respect to the first link to an obtuse angle, when the first link abuts on the annular opening portion; and
a gripping step of gripping the annular opening portion by sandwiching the annular opening portion between the first link and the second link by setting the angle of the second link with respect to the first link to an acute angle, after the second link is inserted into the inside of the annular opening portion.

6. The capturing method according to Claim 5, wherein, in the abutment step, each of the first links is disposed on the same imaginary plane by pivoting each of the first links about the axis and deploying each of the first links to the position spaced away from the supporting body.

7. The capturing method according to Claim 5 or 6, wherein the coupling step includes a centering step of bringing a center position of the supporting body close to a center position of the annular opening portion of the object to be captured by a guide structure having a substantially conical shape formed by each of the second links forming an obtuse angle attitude with respect to the first link.
